# EUROPÄISCHE PATENTSCHRIFT

(11) **EP 0 891 756 B1**
(45) Veröffentlichungstag und Bekanntmachung des Hinweises auf die Patenterteilung: **27.11.2002**
(21) Anmeldenummer: 98890211.0
(22) Anmeldetag: 17.07.1998
(51) Int. Cl.: A61F 2/38

(54) **Gelenk-Prothesenimplantat**
Joint endoprosthesis
Endoprothèse d' articulation

(30) Priorität: 18.07.1997 AT 123397
(43) Veröffentlichungstag der Anmeldung: 20.01.1999
(73) Patentinhaber: Implantech Medizintechnik Ges.m.b.H., 2344 Maria Enzersdorf (AT)
(72) Erfinder: Wurzinger, Anton, Dr., 2344 Maria Enzersdorf (AT); Winter, Franz, 2560 Berndorf (AT)
(74) Vertreter: Kopecky, Helmut, Dipl.-Ing.

(56) Entgegenhaltungen:
- US-A- 5 395 401

## Beschreibung

Die Erfindung betrifft ein Gelenk-Prothesenimplantat, insbesondere für Knieprothesen, das mit einem ersten Implantatteil an einem ersten Knochenende und mit einem zweiten Implantatteil an einem zweiten Knochenende zweier ein Gelenk bildender Knochenenden ansetzbar ist, wobei der erste Implantatteil und der zweite Implantatteil jeweils Gelenkflächen zur Bildung eines Schwenkgelenks aufweisen, das eine Schwenkung um eine etwa quer zur Längserstreckung der die ersten und zweiten Knochenenden aufweisenden ersten und zweiten Knochen gerichtete Schwenkachse ermöglicht, und das weiters einen Gleitteil aufweist, der zusätzlich eine Drehbewegung und eine Verschiebebewegung der Knochenenden zueinander ermöglicht, wobei die Drehbewegung um eine Drehachse etwa quer zur Schwenkachse und etwa in Längsrichtung des ersten Knochens und die Verschiebebewegung in einer Ebene etwa parallel zur Schwenkachse und etwa quer zur Drehachse erfolgt
wobei der erste Implantatteil ein im ersten Knochenende versenkbares Gehäuse aufweist, das in diesem ersten Knochenende verankerbar ist, in dem Gehäuse ein erster Gleitkörper um die Drehachse verdrehbar gelagert ist, der zweite Gleitkörper eine Gelenkfläche trägt, an der der zweite Implantatteil mit der an ihm angeordneten Gelenkfläche anliegt und der zweite Gleitkörper mit einer Gleitfläche an einer Gegengleitfläche, die am Gehäuse vorgesehen ist, aufliegt. Der nächste Stand der Technik stellt das Dokument US-A- 5 395 401 dar.

Die erste Generation brauchbarer Kniegelenke war mit einem Schamiergelenk ausgestattet. Diese für desolate Achsverhältnisse und extreme Bandinstabilitäten zwangsgeführten Achs-Kniegelenke sind heute noch in beschränkter Zahl in Verwendung. Die Zwangsführung behebt zwar die Instabilität, führt aber zu erheblichen Nachteilen, wie erhöhte Materialbelastung und vor allem Lockerung der Implantate aus ihrer Verankerung im Knochen.

Eine nächste große Generation von Kniegelenken besteht aus sogenannten Oberflächen-Prothesen, welche nur neue künstliche Gleitflächen schaffen, wenn der Verschleiß von Knorpel und Menisci kompensiert werden muß, aber der natürliche Band- und Weichteilapparat die Gelenkstabilität aufrecht hält.

Die breite Anwendung von Oberflächen-Prothesen führte zunächst zu einer wesentlichen Verbesserung der femurotibialen Gelenksartikulation. Dieser Prothesentyp räumt dem weitgehend erhaltenen Bandapparat einen hohen Grad von Bewegungsumfang der Femur- und Tibia-Gleitflächen ein. Es handelt sich um sogenannte "flat"-Formen, d.h. möglichst flach ausgebildete Gleitflächen. Diese Entwicklung führte aber gleichzeitig zu empfindlichen Rückschlägen in der Entwicklung künstlicher Knieprothesen. Bei all diesen Prothesendesigns im Bewegungsablauf hat die Femurkufe aus Metall nur eine punktuelle oder linienförmige Auflage auf der Polyäthylen-Gleitfläche der Tibia, einen bis heute unverzichtbaren Implantatwerkstoff. Die punktuelle Überlastung der Polyäthylen-Gleitfläche führt oft zu vorzeitiger Zerstörung von oberflächlichen Gleitpartien sowie auch von Partien, die unterhalb der belasteten Oberfläche liegen. Es kommt zu mehr oder minder erheblich gesteigerten Abriebsverhältnissen, die zur Knochenirritation, Knochenzerstörung und Knochenauflösung (Osteolyse) führen können. In der Gesamtheit kann dieses Abriebsphänomen als Polyäthylen-Krankheit bezeichnet werden, die zur vorzeitigen Dysfunktion der künstlichen Gelenke und des Knochens führen kann.

Die histologischen, tribologischen und klinischen Erkenntnisse leiteten die Entwicklung einer neuen Generation von künstlichen Kniegleitflächen ein. Das Ziel der Entwicklung liegt in der Schaffung neuer Oberflächenstrukturen der Gleitpartner und Verbesserung der Verankerungsverhältnisse, um die frühzeitige Lockerung der Implantate, die durch vermehrten Abrieb und durch Fehlbelastung der Implantate entstehen, aufzuhalten.

Die natürlichen Bewegungsverhältnisse des Kniegelenks sind komplex und durch künstliche Konstruktionen nicht völlig nachvollziehbar. Die dominante Flexions- und Extensionsbewegung, die komplex mit Rotationsbewegungen verbunden sind, deren Rotationszentrum ständig wandert, u. zw. wegen Dislokation des einen Gleitpartners in anteriorer-posteriorer Richtung (AP) durch die sogenannte Roll-Gleitbewegung. Dieser komplexe biomechanische Bewegungsablauf wird ständig von zusätzlichen Faktoren, wie Zuständen des Bandapparates seitlich und in AP-Richtung sowie der Gelenkskapsel, den Muskelzügen, des Patellazuges sowie vor allem der Achsveränderungen der Femur-Tibia-Knochengeometrie beeinflußt.

Das Bestreben der jetzigen Generation von Kniegelenken geht dabei weg von den "flat" (flach)-Formen zu den konformeren Formen, da die geringe Konformität zu überhöhtem "Kontakt-Streß" des Polyäthylens führt, der wiederum erhöhten Abrieb verursacht. Die vergrößerte Konformität andererseits trägt in sich die Gefahr, den Bewegungsablauf einzuschränken. Es sollen schließlich die Rotationsbewegungen und Gleitbewegungen in AP-Richtung nicht allzu stark von den natürlichen Verhältnissen abweichen. Das heutige Bestreben besteht in der Erreichung einer hohen Konformität ohne Einschränkung der Rotations- und Gleitbewegungen.

Es gibt bereits funktionierende Kniegelenke (US-A 4 340 978 und US-A 4 309 778), die einerseits die Rotationsbewegung, sogenannte "rotating-platform" zulassen und andererseits "künstliche Menisci", sogenannte "meniscal-bearing", die auf einer Art Schienenführung in AP-Richtung fahren und die AP-Dislokation nachahmen. Die aus den beiden oben zitierten US-Patentschriften bekannten Kniegelenke sind insofern kompliziert im Aufbau, als zwischen dem ersten und dem zweiten Implantatteil am ersten Implantatteil zwei im Abstand voneinander angeordnete und am ersten Implantatteil in Schienen geführte und die Gelenkflächen zur Bildung des Schwenkgelenks bildende Gleitstücke eingesetzt sind, welche Gleitstücke sowohl eine Drehbewegung als auch eine Schwenkbewegung ermöglichen, zumal die Schienenführungen bogenförmig angeordnet sind. Dies hat jedoch den Nachteil, daß die Schienenführungen zum Gelenk hin völlig frei und offen liegen und daß die Drehbewegungen nur genau nach den Schienenführungen durchführbar sind, was jedoch zur Folge hat, daß eine Verschiebebewegung nicht mehr möglich ist. Nach einer anderen Ausführungsform gemäß den US-Patentschriften weisen die Schienenführungen jedoch ein sehr großes Spiel auf, so daß nicht nur Drehbewegungen, sondern auch in sehr beschränktem Ausmaß Verschiebebewegungen möglich sind, wodurch jedoch die Drehbewegungen völlig unkontrolliert und nicht an einer Drehachse orientiert sind. Eine Kombination beider Bewegungsabläufe, nämlich der Drehbewegung und der Verschiebebewegung, konnte bisher nicht befriedigend gelöst werden, da die rotierende Plattform nicht wandert und die schienengeführte Meniscal-Bewegung nicht oder kaum rotieren kann. Insbesondere ist es bisher nicht möglich, Verschiebebewegungen unabhängig von der Drehbewegung, d.h. in jeder Drehlage durchzuführen.

Die Erfindung bezweckt die Vermeidung dieser Nachteile und Schwierigkeiten und stellt sich die Aufgabe, ein Gelenk-Prothesenimplantat der eingangs beschriebenen Art zu schaffen, bei dem jedoch eine Kombination sowohl der Drehbewegung als auch der Verschiebebewegung durchführbar ist, derart, daß die Drehbewegung kontrolliert auch um größere Beträge stattfinden kann und gleichzeitig eine Gleitbewegung ebenfalls in kontrolliertem Umfang durchgeführt werden kann, u.zw. unabhängig von der Drehbewegung. Hierdurch sollen die Verankerungspartien des Gelenk-Prothesenimplantats geschont und eine lange Haltbarkeit der Verankerung erzielt werden. Weiters sollen alle Druck-, Zug-, Seiten- und Schubkräfte großflächig übertragbar und neutralisierbar sein; Spitzenbelastungen sollen verhindert werden.

Diese Aufgabe wird erfindungsgemäß dadurch gelöst,
- daß in dem ersten Gleitkörper der zweite Gleitkörper in einer Richtung etwa quer zur Drehachse verschiebbar gelagert ist,
- daß der zweite Gleitkörper aus dem Gehäuse herausragt, und
- daß die Gelenkfläche des zweiten Gleitkörpers an dem aus dem Gehäuse herausragenden Teil getragen wird.

Hierbei ist vorteilhaft der erste Gleitkörper zur Gänze vom Gehäuse seitlich umschlossen und durch eine vom ersten Knochen abgewendete Öffnung des Gehäuses in das Gehäuse einsetzbar bzw. aus dem Gehäuse entfernbar.

Zur Stabilisierung des Gelenk-Prothesenimplantats ist vorteilhaft der erste Gleitkörper in Richtung der Längserstreckung des ersten Knochens im Gehäuse in Richtung der Drehachse gegen Entfernen aus dem Gehäuse gesichert, u.zw. zweckrnäßig mittels einer bajonettartigen Einrichtung, die vorteilhaft dadurch gekennzeichnet ist, daß zur axialen Sicherung zwei sich quer durch das Gehäuse erstreckende und etwa parallel zueinander gerichtete Stifte vorgesehen sind, die sich in einer zur Schwenkachse etwa parallelen Richtung erstrecken und die von einem am ersten Gleitkörper angeordneten Fußteil untergriffen sind, wobei der Fußteil in einer Richtung lediglich eine maximale Breite aufweist, die der Distanz) der beiden Stifte entspricht und in einer dazu um etwa 90° stehenden Richtung die Stifte hintergreifende Fortsätze aufweist.

Eine bevorzugte Ausführungsform ist dadurch gekennzeichnet, daß daß der erste Gleitkörper mit einer zur Öffnung des Gehäuses offenen hinterschnittenen Nut versehen ist, die von zueinander parallel ausgerichteten Seitenwänden gebildet ist, welche Nut sich in Richtung der Verschiebbarkeit des zweiten Gleitkörpers gegenüber dem ersten Gleitkörper erstreckt, und wobei der zweite Gleitkörper mit einer korrespondierend zur Nut ausgebildeten Feder versehen ist und damit in die Nut eingreift, wobei zweckmäßig sich die Nut quer durch den gesamten ersten Gleitkörper erstreckt und die Feder des zweiten Gleitkörpers eine um den Verschiebeweg geringere Erstreckung in Richtung der Verschiebbarkeit des zweiten Gleitkörpers gegenüber dem ersten Gleitkörper aufweist als die Nut.

Der erste Gleitkörper ist aus Festigkeitsgründen vorteilhaft aus Metall gebildet.

Eine bevorzugte Ausführungsform ist dadurch gekennzeichnet, daß das Gehäuse einen sich von der Öffnung des Innenraums nach innen erstreckenden zylindrischen Teil des Innenraums und einen zum Boden des Gehäuses daran anschließenden konischen Teil, insbesondere kegelstumpfförmigen Teil, aufweist, wobei vorteilhaft das Gehäuse an seinem sich in den Knochen erstreckenden Ende einen Stützanker zum Einsetzen in den Markraum aufweist, an dem vorzugsweise ein Verlängerungsanker befestigt werden kann.

Aus Festigkeitsgründen ist das Gehäuse ebenfalls aus Metall gebildet.

Um ein gutes Gleiten des zweiten Gleitkörpers am Gehäuse sicherzustellen, ist vorteilhaft das Gehäuse an der dem ersten Knochen abgewendeten Seite mit einer radial vom Gehäuse nach außen vorstehenden Gleitplatte versehen, wobei zweckmäßig der zweite Gleitkörper mit einer Gleitplatte versehen ist, die etwa gleich bemessen ist wie das Gehäuse und mit dieser Gleitplatte an der Gleitplatte des Gehäuses aufliegt.

Zur Anpassung an die Knochenkontur und zum Zweck des hindernisfreien Führens eines Bandes, wie z.B. eines Kreuzbandes, bei Verwendung des Gelenk-Prothesenimplantats als Kniegelenk sind zweckmäßig sowohl die Gleitplatte des Gehäuses als auch die Gleitplatte des zweiten Gelenkkörpers nierenförmig gestaltet, wobei sich die Längserstreckung der Gleitplatte des Gehäuses etwa in Richtung der Schwenkachse erstreckt.

Der zweite Gleitkörper ist vorzugsweise aus Kunststoff gefertigt, insbesondere aus Polyäthylen.

Die vorzugsweise Verwendung des Gelenk-Prothesenimplantats ist die als Kniegelenk. Hierbei ist das Gehäuse im Tibiakopf verankerbar.

Zwecks optimaler Verankerung des ersten Implantatteils im Knochen weist dieser an seinen knochenseitigen Außenseiten strukturierte Oberflächen auf, wobei insbesondere der erste Implantatteil an der Seite der Gleitplatte, an der diese am Knochenende aufliegt, eine Vielzahl zylindrischer Vertiefungen, insbesondere Bohrungen mit Durchmessern von 0,3 bis 1 mm, vorzugsweise 0,5 bis 1 mm, und mit Tiefen zwischen 0,3 und 1 mm, vorzugsweise 0,5 und 1 mm, aufweist, so daß eine Porosität zwischen 50 und 80 %, vorzugsweise 60 und 70 %, bewirkt ist. Vorzugsweise sind zusätzlich zu den zylindrischen Vertiefungen zylindrische Erhebungen mit Durchmessern zwischen 0,3 und 1 mm, vorzugsweise 0,5 und 1 mm, und Höhen zwischen 0,3 und 1 mm, vorzugsweise 0,5 und 1 mm vorhanden, wobei das Verhältnis der Anzahl der zylindrischen Erhebungen zur Anzahl der zylindrischen Vertiefungen 3 : 7 beträgt und eine Porosität zwischen 50 und 80 %, vorzugsweise 60 und 70 %, vorhanden ist..

An den nicht-planen Flächen des ersten Implantatteils, mit denen er mit dem Knochen in Berührung steht, sind kraterförmig strukturierte Vertiefungen mit Tiefen bis zu 0,5 mm vorgesehen.

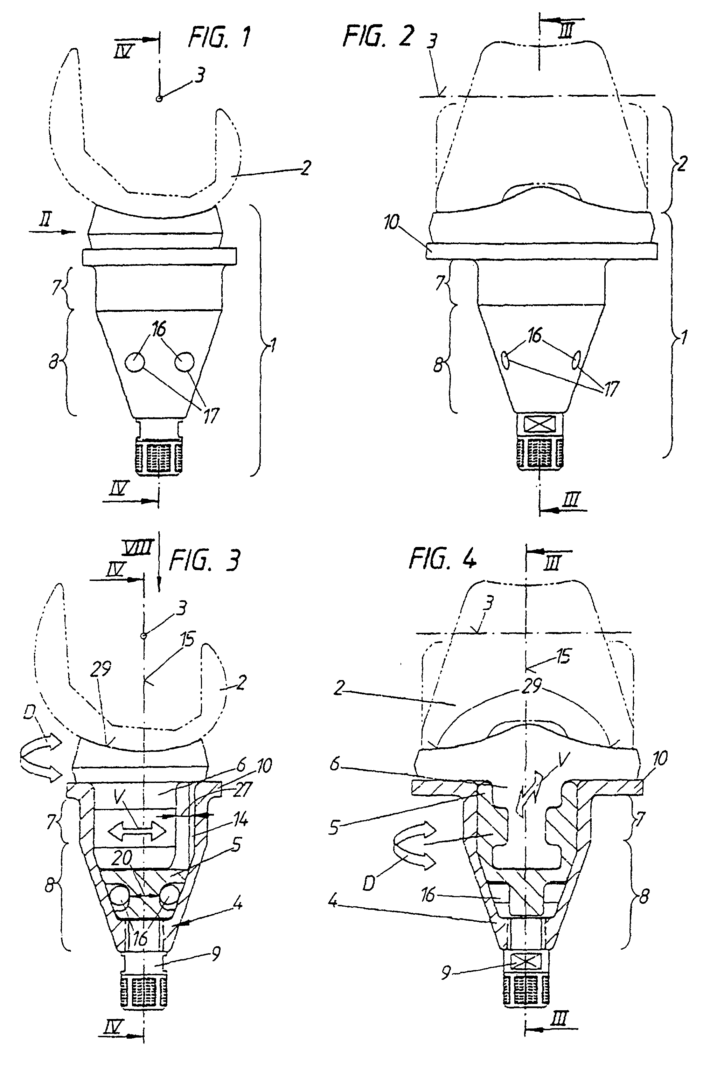

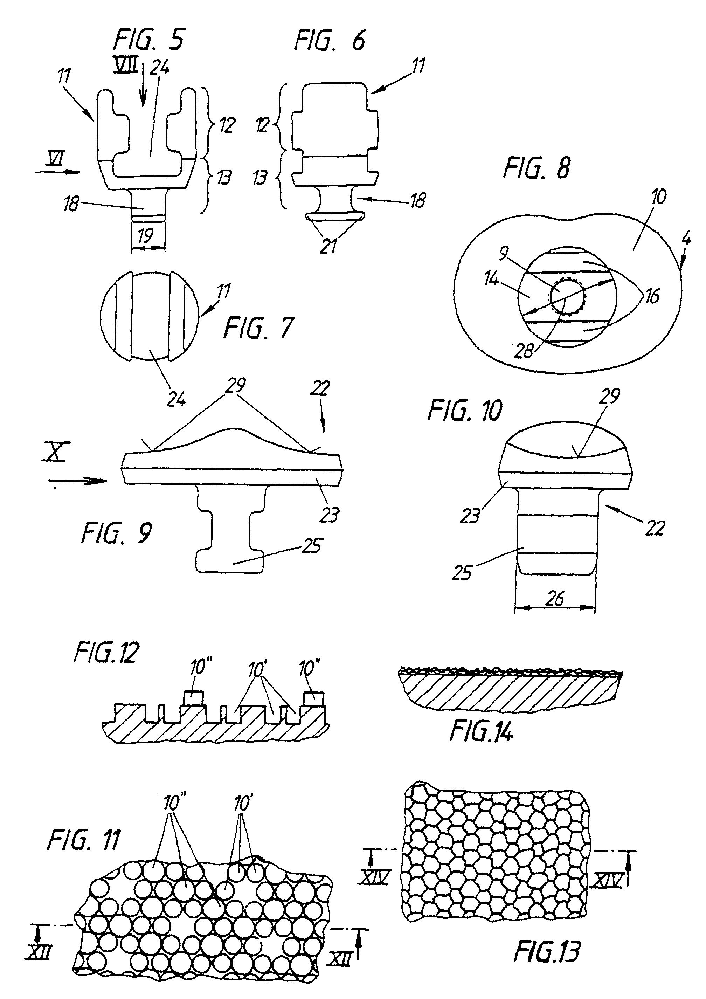

Die Erfindung ist nachfolgend anhand eines in der Zeichnung dargestellten Ausführungsbeipiels näher erläutert, wobei die Fig. 1 und 2 jeweils Seitenansichten des Gelenk-Prothesenimplantats, u.zw. Fig. 2 gemäß dem Pfeil II der Fig. 1, und die Fig. 3 und 4 Schnitte durch den ersten Implantatteil, u.zw. Fig. 3 entlang der Linie III-III der Fig. 2 und Fig. 4 entlang der Linie IV-IV der Fig. 1 zeigen. Fig. 5 stellt eine Seitenansicht des ersten Gleitkörpers, Fig. 6 eine Ansicht desselben in Richtung des Pfeiles VI der Fig. 5 und Fig. 7 eine Draufsicht auf denselben in Richtung des Pfeiles VII der Fig. 5 dar. Fig 8 veranschaulicht eine Ansicht des Gehäuses in Richtung des Pfeiles VIII der Fig. 3. Fig. 9 zeigt eine Seitenansicht des zweiten Gleitkörpers, Fig. 10 eine Ansicht in Richtung des Pfeiles X der Fig. 9. In den Fig. 11 und 12 sowie 13 und 14 sind jeweils Oberflächenstrukturen in Draufsicht und im Schnitt nach den Linien XII-XII und XIV-XIV der Fig. 11 und 13 veranschaulicht.

Das erfindungsgemäße Gelenk-Prothesenimplantat, das in der Zeichnung dargestellt ist, ist als Knieprothese ausgebildet. Es könnte jedoch mit Modifikationen auch für andere Gelenke eingesetzt werden. Es weist einen ersten Implantatteil 1 auf, der im Tibiakopf verankerbar ist, entweder zementlos oder durch Einzementieren. Er bildet mit einem zweiten Implantatteil 2, der am Femur befestigbar ist, ein Schwenkgelenk, das eine Schwenkmöglichkeit um eine Schwenkachse 3 ermöglicht, die der Schwenkung des Unterschenkels gegenüber dem Oberschenkel entspricht.

Der erste Implantatteil 1 ist im wesentlichen von drei Teilen gebildet, nämlich einem Gehäuse 4, einem ersten und einem zweiten Gleitkörper 5 und 6. Das Gehäuse, das in etwa eine gleichmäßige Wandstärke aufweist, weist einen zylindrischen Teil 7 und einen an diesen anschließenden kegelstumpfförmigen Teil 8 auf, der in einen Stützanker 9 übergeht. Das Gehäuse ist 4 entweder integral oder eingeschraubt mit seinen zylindrischen und kegelstumpfförmigen Teilen 7, 8 zur Gänze in den Tibiakopf einsetzbar, wobei der Stützanker 9 in den Markraum ragt. An den Stützanker 9 kann gegebenenfalls ein Verlängerungsanker mit geeigneter Länge befestigt werden, sollte dies aufgrund des Knochenaufbaus erforderlich sein.

Am oberen Ende des Gehäuses 4, d.h. an dem Ende, das dem Tibiakopf abgewendet ist, weist das Gehäuse 4 eine radial vom Gehäuse 4 nach außen kragende Gleitplatte 10 mit etwa nierenförmiger Flächenerstreckung auf. Die Längserstreckung dieser nierenförmigen Gleitplatte ist in etwa parallel zur Schwenkachse 3 ausgerichtet. Die von der Tibia freiliegende Seite der Gleitplatte 10 ist poliert, die Unterseite der Gleitplatte, die am Tibiakopf aufliegt, weist eine strukturierte Oberfläche auf, wie in den Fig. 11 und 12 dargestellt, u.zw. wird die Strukturierung durch eine Vielzahl zylindrischer Bohrungen 10' mit Durchmessern von 0,3 bis 1 mm, vorzugsweise 0,5 bis 1 mm, und mit Tiefen zwischen 0,3 und 1 mm, vorzugsweise 0,5 und 1 mm, erreicht. Weiters sind noch zylindrische Erhebungen 10" vorhanden, u.zw. mit Durchmessern zwischen 0,3 und 1 mm, vorzugsweise 0,5 und 1 mm, und Höhen zwischen 0,3 und 1 mm, vorzugsweise 0,5 und 1 mm, wobei das Verhältnis der Anzahl der zylindrischen Erhebungen 10" zur Anzahl der zylindrischen Bohrungen 10' 3 : 7 beträgt. Insgesamt wird hierdurch eine Porosität zwischen 50 und 80, vorzugsweise 60 und 70 %, bewirkt. Als Porosität ist der Prozentsatz des Luftvolumens über einem Oberflächenbereich, der mit Bohrungen bzw. Erhebungen versehen ist, gegenüber dem massiven Volumen dieses Oberflächenbereiches zu verstehen.

An den nicht-planen Flächen, mit denen das Gehäuse 4 mit dem Knochen in Berührung steht, sind kraterförmige Vertiefungen mit Tiefen bis zu 0,5 mm vorgesehen, wie dies in den Fig. 13 und 14 dargestellt ist.

Das Gehäuse 4 ist aus Metall gebildet; insbesondere können hier folgende Metalle zum Einsatz gelangen: Reintitan, eine Titanguß- oder Titanschmiede-Legierung oder eine Kobalt-Chrom-Legierung.

In das Gehäuse 4 ist ein erster Gleitteil 11 eingesetzt, der einen zylindrischen und einen kegelstumpfförmigen Teil 12 bzw. 13 aufweist und mit diesen Teilen in die zylindrische und kegelstumpfförmige Innenausnehmung 14 des Gehäuses 4 mit geringem Spiel paßt, so daß der erste Gleitteil 11 gegenüber dem Gehäuse 4 drehbar ist, u.zw. um eine Drehachse 15, die etwa parallel zur Längsachse der Tibia gerichtet ist.

Dieser erste Gleitteil paßt zur Gänze in die Innenausnehmung 14 des Gehäuses 4 und ist gegen Herausfallen aus dem Gehäuse 4 durch eine Art Bajonettverriegelung gesichert. Diese bajonettartige Einrichtung ist durch zwei sich quer durch die Innenausnehmung 14 des Gehäuses, u.zw. durch die kegelstumpfförmige Ausnehmung des Gehäuses erstreckende Stifte 16, die in entsprechende Bohrungen 17 des Gehäuses 4 eingesetzt sind und in diesen Bohrungen 14 fixiert sind, beispielsweise durch einen Preßsitz, verwirklicht. Die Längsachsen der Stifte liegen symmetrisch zur Drehachse 15 und in seitlichem Abstand von dieser. Der erste Gleitkörper 11 weist an seinem unteren Ende einen Fußteil 18 auf, der in einer Richtung eine maximale Breite 19 aufweist, die der Distanz 20 der beiden Stifte 16 voneinander entspricht. In einer dazu um etwa 90° gedrehten Richtung weist der Fußteil 18 die Stifte 16 hintergreifende Fortsätze 21 auf. Auf diese Art und Weise ist der erste Gleitteil 11 in das Gehäuse 4 nur in einer ganz bestimmten Drehstellung des ersten Gleitkörpers 11 zum Gehäuse 4 einsetzbar, u.zw. dann, wenn der Fußteil 18 mit seinen Fortsätzen 21 etwa parallel ausgerichtet ist zu den Stiften 16. Nach einer Drehung um einen bestimmten Mindestwinkel hintergreifen die Fortsätze 21 die Stifte 16 und der erste Gleitteil 11 kann nicht mehr durch eine Bewegung längs der Drehachse 15 aus dem Gehäuse 4 entfernt werden. Der erste Gleitkörper ist ebenfalls wie das Gehäuse aus Metall gebildet.

Der erste Gleitkörper 11 dient zur Aufnahme eines zweiten Gleitkörpers 22, der gegenüber dem ersten Gleitkörper 11, und damit auch gegenüber dem Gehäuse 4 verschiebbar ist, u.zw. in einer Ebene, die etwa parallel gerichtet ist zur Schwenkachse 3 und senkrecht zur Drehachse 15. Der zweite Gleitkörper 22 weist ebenfalls eine Gleitplatte 23 auf, die in ihrer Umfangsgestaltung der am Gehäuse 4 angeordneten Gleitplatte 10 entspricht, also auch nierenförmig gestaltet ist. Bei in das Gehäuse 4 eingesetztem zweiten Gleitteil 22 liegt die Gleitplatte 23 des zweiten Gleitteils 22 auf der Gleitplatte 10 des Gehäuses 4 auf. Damit jedoch bei Verschiebungen nicht die Gleitplatte 23 des zweiten Gleitkörpers 22 über die Gleitplatte 10 des Gehäuses 4 vorragt, ist die Gleitplatte 23 des zweiten Gleitkörpers 22 in ihrer Flächenerstreckung etwas geringer dimensioniert als die Gleitplatte 10 des Gehäuses 4.

Der zweite Gleitkörper 22 ist gegenüber dem ersten Gleitkörper 11 nicht nur verschiebbar gelagert, sondern gegenüber diesem auch gegen ein axiales Entfernen, u.zw. in Richtung der Drehachse 15 gesichert. Diese beiden Funktionen werden durch eine im ersten Gleitkörper 11 vorgesehene hinterschnittene Nut 24 erfüllt, zu der der zweite Gleitkörper eine korrespondierend ausgebildete und mit der Gleitplatte 23 integral ausgebildete Feder 25 aufweist, mit der er in die Nut 24 eingesetzt ist. Der Verschiebeweg des zweiten Gleitkörpers 22 gegenüber dem Gehäuse 4 wird durch die Längserstreckung 26 der Feder 25 definiert. Die Längserstreckung 26 der Feder 25 ist genau um den gewünschten Verschiebeweg 27 kürzer bemessen als der Innendurchmesser 28 des Innenraums 14 des Gehäuses 4 (vgl. Fig. 3 und 8). Der zweite Gleitkörper 22 kann somit immer nur vom Anschlag der Feder 25 an der einen Seite des Gehäuses 4 bis zum Anschlag der Feder 25 an der anderen Seite des Gehäuses 4 bewegt werden.

Der zweite Gleitkörper 22, der aus Kunststoff, vorzugsweise aus Polyäthylen, gefertigt ist, weist an der oberen Seite der Gleitplatte 23, d.h. der vom Gehäuse 4 abgewendeten Seite Gelenkpfannen 29 für den zweiten Implantatteil 2, der mit dem Femur verbunden ist, auf.

Die Funktion des Gelenk-Prothesenimplantats ist nachfolgend näher erläutert:

Das erfindungsgemäße Gelenk-Prothesenimplantat trägt den natürlichen multizentrischen Bewegungsabläufen Rechnung, u.zw. in stark verbesserter Form als die bisherigen Gelenk-Prothesenimplantate. Die erfindungsgemäße Bewegung, die durch das Gelenk-Prothesenimplantat ermöglicht wird, besteht darin, daß in der anterior-posterioren Dislokationsbewegung, die der natürlichen Gleitbewegung nachempfunden ist, gleichzeitig auf all ihren Bewegungspunkten jeweils ein neues Rotationszentrum entsteht und darum gleichzeitig auf allen diesen Gleitpunkten die Rotation nach medial und lateral stattfinden kann. Diese sogenannte "Multiplex-Bewegung", die mit ihrer Dislokationsstrecke auf dem Bewegungssektor von Beugung und Streckung seiner Hauptbelastung des Gehens wirksam wird, hat die Rotationsfreiheit, welche von den Bedürfnissen des Weichteilapparates, der Knochen-Achsgeometrie und anderer individueller Erfordernisse, kurz, in der Summe der komplexen Anforderungen, die für das jeweilige individuelle Kniegelenk erforderlich ist. Aus diesem Multiplex-Bewegungsprinzip resultiert die Ausstattungsmöglichkeit der Tibia-Gleitflächen mit einer hohen Konformität der Gelenkpartner von Femur-Implantatteil 2 und Tibia-Implantatteil 1, wie sie bisher nicht möglich war. Die hohe Konformität der Polyäthylen-Gleitfläche des Gleitkörpers 22 läßt ein Maximum an Verteilung von Druck- und Schiebekräften zwischen dem Femur-Implantatteil 2 und dem Gleitkörper 22 entstehen. In besonderer Weise wird die Streßbelastung der Polyäthylen-Gleitfläche des Gleitkörpers 22, die durch die Roll-Gleitbewegung der AP-Dislokation steht, erheblich reduziert. Die Multi-Rotationsbewegungen auf allen Punkten der Dislokationsbahn der Tibia-Gleitfläche des Gleitkörpers 22 fangen weiters die seitlichen Streß-Bewegungen und Schub-Bewegungen auf, die sonst voll zu Lasten der Tibiaverankerung gehen.

Das neue Multiplex-Bewegungs-Gleitprinzip führt zusammenfassend zu drei wesentlichen Verbesserungen des künstlichen Kniegelenkes:
1. Höhere Konformität der Artikulationsfläche von Femur und Tibia.
2. Reduzierung der Streß-Belastung der Polyäthylen-Tibia-Gleitfläche.
3. Der multiforme Bewegungsablauf schont die Verankerungspartien.

Die Funktion der Gleichzeitigkeit der Bewegung von Rotation nach medial und lateral sowie der Dislokationsbewegung von anterior-posterior ist für die Erfindung wesentlich. Diese Funktion wird auf den zweiten Gleitkörper 22 übertragen. Alle Druck-, Zug-, Seiten- und Schubkräfte werden großflächig, d.h. bei maximaler Auflage des Femurteils, d.h. des zweiten Implantatteiles 2, auf den zweiten Gleitkörper 22 der Tibia übertragen und durch das erfindungsgemäße Multiplex-Bewegungsprinzip neutralisiert. Diese Kraftverteilung verhindert punktuelle oder kleinflächige fokussierende Streßbelastung auf das Polyäthylen und verhindert gleichzeitig Spitzenbelastungen auf die Verankerung der Implantate 1, 2 von Tibia wie Femur.

Das feinpolierte tischartige Gleitlager des zweiten Gleitkörpers 22, das mit dem zweiten Femur-Implantatteil 2 konform korrespondiert, erlaubt die notwendige Roll- und Gleitbewegung in Beugung und Streckung des Gelenkes bei maximalem Flächenkontakt, wird aber seitlich durch die Konformität festgehalten. Der zweite Gleitkörper 22 selbst bewegt sich großflächig auf der Gleitplatte 10 des Gehäuses 4. Die Kräfte werden also großflächig von der Unterseite des zweiten Gleitkörpers 22 auf die große Gleitplatte 10 des Gehäuses 4 übertragen und gehen nicht zu Lasten der tiefer liegenden Partien, die lediglich eine Führungs- und Haltefunktion ausüben. Das neue erfindungsgemäße Multiplex-Bewegungsprinzip strebt ein Minimum an Materialbelastung vor allem des Tibia-Implantatteiles 1 an. Ein Ziel, das durch diese Erfindung erreicht wird, besteht in der Verminderung der Polyäthylen-Krankheit, ein anderes, ebenfalls erreichtes Ziel besteht in der Schonung der Verankerung zur Verhinderung der vorzeitigen Lockerung der Implantate, die deren Lebensdauer begrenzt.

## Patentansprüche

1. Gelenk-Prothesenimplantat, insbesondere für Knieprothesen, das mit einem ersten Implantatteil (1) an einem ersten Knochenende und mit einem zweiten Implantatteil (2) an einem zweiten Knochenende zweier ein Gelenk bildender Knochenenden ansetzbar ist, wobei der erste Implantatteil (1) und der zweite Implantatteil (2) jeweils Gelenkflächen zur Bildung eines Schwenkgelenks aufweisen, das eine Schwenkung um eine etwa quer zur Längserstreckung der die ersten und zweiten Knochenenden aufweisenden ersten und zweiten Knochen gerichtete Schwenkachse (3) ermöglicht, und das weiters einen Gleitteil (11, 22) aufweist, der zusätzlich eine Drehbewegung (D) und eine Verschiebebewegung (V) der Knochenenden zueinander ermöglicht, wobei die Drehbewegung (D) um eine Drehachse (15) etwa quer zur Schwenkachse (3) und etwa in Längsrichtung des ersten Knochens und die Verschiebebewegung (V) in einer Ebene etwa parallel zur Schwenkachse (3) und etwa quer zur Drehachse (15) erfolgt, wobei der erste Implantatteil (1) ein im ersten Knochenende versenkbares Gehäuse (4) aufweist, das in diesem ersten Knochenende verankerbar ist, in dem Gehäuse (4) ein erster Gleitkörper (11) um die Drehachse (15) verdrehbar gelagert ist, der zweite Gleitkörper (22) eine Gelenkfläche (29) trägt, an der der zweite Implantatteil (2) mit der an ihm angeordneten Gelenkfläche anliegt und der zweite Gleitkörper (22) mit einer Gleitfläche an einer Gegengleitfläche, die am Gehäuse (4) vorgesehen ist, aufliegt, **dadurch gekennzeichnet,**
- **dass** in dem ersten Gleitkörper (11) der zweite Gleitkörper (12) in einer Richtung etwa quer zur Drehachse (15) verschiebbar gelagert ist,
- **dass** der zweite Gleitkörper (22) aus dem Gehäuse (4) herausragt und
- **dass** die Gelenkfläche (29) des zweiten Gleitkörpers (22) an dem aus dem Gehäuse (4) herausragenden Teil getragen wird.

2. Gelenk-Prothesenimplantat nach Anspruch 1, **dadurch gekennzeichnet, daß** der erste Gleitkörper (11) zur Gänze vom Gehäuse (4) seitlich umschlossen ist und durch eine vom ersten Knochen abgewendete Öffnung des Gehäuses (4) in das Gehäuse (4) einsetzbar bzw. aus dem Gehäuse (4) entfernbar ist.

3. Gelenk-Prothesenimplantat nach Anspruch 1 oder 2, **dadurch gekennzeichnet, daß** der erste Gleitkörper (11) in Richtung der Längserstreckung des ersten Knochens im Gehäuse (4) in Richtung der Drehachse (15) gegen Entfernen aus dem Gehäuse (4) gesichert ist.

4. Gelenk-Prothesenimplantat nach Anspruch 3, **dadurch gekennzeichnet, daß** die axiale Sicherung mittels einer bajonettartigen Einrichtung (16, 18) verwirklicht ist.

5. Gelenk-Prothesenimplantat nach Anspruch 4, **dadurch gekennzeichnet, daß** zur axialen Sicherung zwei sich quer durch das Gehäuse (4) erstreckende und etwa parallel zueinander gerichtete Stifte (16) vorgesehen sind, die sich in einer zur Schwenkachse (3) etwa parallelen Richtung erstrecken und die von einem am ersten Gleitkörper (11) angeordneten Fußteil (18) untergriffen sind, wobei der Fußteil in einer Richtung lediglich eine maximale Breite (19) aufweist, die der Distanz (20) der beiden Stifte (16) entspricht und in einer dazu um etwa 90° stehenden Richtung die Stifte (16) hintergreifende Fortsätze (21) aufweist.

6. Gelenk-Prothesenimplantat nach einem oder mehreren der Ansprüche 1 bis 5, **dadurch gekennzeichnet, daß** der erste Gleitkörper (11) mit einer zur Öffnung des Gehäuses (4) offenen hinterschnittenen Nut (24) versehen ist, die von zueinander parallel ausgerichteten Seitenwänden gebildet ist, welche Nut (24) sich in Richtung der Verschiebbarkeit des zweiten Gleitkörpers (22) gegenüber dem ersten Gleitkörper (11) erstreckt, und wobei der zweite Gleitkörper (22) mit einer korrespondierend zur Nut (24) ausgebildeten Feder (25) versehen ist und damit in die Nut (24) eingreift.

7. Gelenk-Prothesenimplantat nach Anspruch 6, **dadurch gekennzeichnet, daß** sich die Nut (24) quer durch den gesamten ersten Gleitkörper (11) erstreckt und daß die Feder (25) des zweiten Gleitkörpers (22) eine um den Verschiebeweg (27) geringere Erstreckung in Richtung der Verschiebbarkeit des zweiten Gleitkörpers (22) gegenüber dem ersten Gleitkörper (11) aufweist als die Nut (24).

8. Gelenk-Prothesenimplantat nach einem oder mehreren der Ansprüche 1 bis 7, **dadurch gekennzeichnet, daß** der erste Gleitkörper (11) aus Metall gebildet ist.

9. Gelenk-Prothesenimplantat nach einem oder mehreren der Ansprüche 1 bis 8, **dadurch gekennzeichnet, daß** das Gehäuse (4) einen sich von der Öffnung des Innenraums nach innen erstreckenden zylindrischen Teil (7) des Innenraums und einen zum Boden des Gehäuses daran anschließenden konischen Teil (8), insbesondere kegelstumpfförmigen Teil (8), aufweist.

10. Gelenk-Prothesenimplantat nach einem oder mehreren der Ansprüche 1 bis 9, **dadurch gekennzeichnet, daß** das Gehäuse (4) an seinem sich in den Knochen erstreckenden Ende einen Stützanker (9) zum Einsetzen in den Markraum aufweist, an dem vorzugsweise ein Verlängerungsanker befestigt werden kann.

11. Gelenk-Prothesenimplantat nach einem oder mehreren der Ansprüche 1 bis 10, **dadurch gekennzeichnet, daß** das Gehäuse (4) aus Metall gebildet ist.

12. Gelenk-Prothesenimplantat nach einem oder mehreren der Ansprüche 1 bis 11, **dadurch gekennzeichnet, daß** das Gehäuse (4) an der dem ersten Knochen abgewendeten Seite mit einer radial vom Gehäuse nach außen vorstehenden Gleitplatte (10) versehen ist.

13. Gelenk-Prothesenimplantat nach Anspruch 12, **dadurch gekennzeichnet, daß** der zweite Gleitkörper (22) mit einer Gleitplatte (23) versehen ist, die etwa gleich bemessen ist wie das Gehäuse (4) und mit dieser Gleitplatte (23) an der Gleitplatte (10) des Gehäuses (4) aufliegt.

14. Gelenk-Prothesenimplantat nach Anspruch 12 oder 13, **dadurch gekennzeichnet, daß** sowohl die Gleitplatte (10) des Gehäuses (4) als auch die Gleitplatte (23) des zweiten Gelenkkörpers (22) nierenförmig gestaltet sind, wobei sich die Längserstreckung der Gleitplatte (10) des Gehäuses (4) etwa in Richtung der Schwenkachse (3) erstreckt.

15. Gelenk-Prothesenimplantat nach einem oder mehreren der Ansprüche 1 bis 14, **dadurch gekennzeichnet, daß** der zweite Gleitkörper (22) aus Kunststoff gefertigt ist, insbesondere aus Polyäthylen.

16. Gelenk-Prothesenimplantat nach einem oder mehreren der Ansprüche 1 bis 15, **dadurch gekennzeichnet, daß** das Gehäuse (4) im Tibiakopf verankerbar ist.

17. Gelenk-Prothesenimplantat nach einem oder mehreren der Ansprüche 1 bis 16, **dadurch gekennzeichnet, daß** der erste Implantatteil (1) an seinen knochenseitigen Außenseiten strukturierte Oberflächen aufweist.

18. Gelenk-Prothesenimplantat nach Anspruch 17, **dadurch gekennzeichnet, daß** der erste Implantatteil an der Seite der Gleitplatte, an der diese am Knochenende aufliegt, eine Vielzahl zylindrischer Vertiefungen (10'), insbesondere Bohrungen (10'), mit Durchmessern von 0,3 bis 1 mm, vorzugsweise 0,5 bis 1 mm, und mit Tiefen zwischen 0,3 und 1 mm, vorzugsweise 0,5 und 1 mm, aufweist, so daß eine Porosität zwischen 50 und 80 %, vorzugsweise 60 und 70 %, bewirkt ist (Fig. 11, 12).

19. Gelenk-Prothesenimplantat nach Anspruch 18, **dadurch gekennzeichnet, daß** zusätzlich zu den zylindrischen Vertiefungen zylindrische Erhebungen (10") vorhanden sind mit Durchmessern zwischen 0,3 und 1 mm, vorzugsweise 0,5 und 1 mm, und Höhen zwischen 0,3 und 1 mm, vorzugsweise 0,5 und 1 mm, wobei das Verhältnis der Anzahl der zylindrischen Erhebungen (10") zur Anzahl der zylindrischen Vertiefungen (10') 3 : 7 beträgt und eine Porosität zwischen 50 und 80 %, vorzugsweise 60 und 70 %, vorhanden ist (Fig. 11, 12).

20. Gelenk-Prothesenimplantat nach einem oder mehreren der Ansprüche 17 bis 19, **dadurch gekennzeichnet, daß** der erste Implantatteil (1) an den nicht-planen Flächen, mit denen er mit dem Knochen in Berührung steht, kraterförmig strukturierte Vertiefungen mit Tiefen bis zu 0,5 mm aufweist (Fig. 13, 14).

## Claims

1. Joint endoprosthesis, in particular for knee prostheses, which by a first implant component (1) can be attached to a first epiphysis and by a second implant component (2) to a second epiphysis of two epiphyses that form a joint, wherein the first implant component (1) and the second implant component (2) each have articulation surfaces for the formation of a swivel joint enabling swiveling about a swivel axis (3) directed roughly transversally with respect to the longitudinal extent of the first and second bones that have the first and second epiphyses, and which furthermore has a sliding piece (11, 22) which additionally enables a rotational movement (D) and a translational movement (V) of the epiphyses relative to each other, with the rotational movement (D) being about an axis of rotation (15) roughly transversal to the swivel axis (3) and roughly in the longitudinal direction of the first bone and the translational movement (V) being in a plane that is roughly parallel to the swivel axis (3) and roughly transversal with respect to the axis of rotation (15), wherein the first implant component (1) has a housing (4) that can be embedded in the first epiphysis and anchored in that first epiphysis, in the housing (4) a first sliding body (11) is mounted so as to be rotatable about the axis of rotation (15), the second sliding body (22) carries an articulation surface (29) against which the second implant component (2) rests by the articulation surface arranged thereupon and the second sliding body (22) by a sliding surface rests against a mating sliding surface provided on the housing (4), **characterized in**
- **that** in the first sliding body (11) the second sliding body (12) is mounted so as to be translatable in a direction roughly transversal to the axis of rotation (15),
- **that** the second sliding body (22) projects out of the housing (4), and
- **that** the articulation surface (29) of the second sliding body (22) is supported at the portion that projects out of the housing (4).

2. Joint endoprosthesis according to claim 1, **characterized in that** the first sliding body (11) laterally is completely surrounded by the housing (4) and through an opening of the housing (4) that faces away from the first bone can be inserted into the housing (4) or removed from the housing (4).

3. Joint endoprosthesis according to claim 1 or 2, **characterized in that** the first sliding body (11) in the direction of the longitudinal extent of the first bone in the housing (4) in the direction of the axis of rotation (15) is secured against removal from the housing (4).

4. Joint endoprosthesis according to claim 3, **characterized in that** axial retainment is realized by means of a bayonet-type device (16, 18).

5. Joint endoprosthesis according to claim 4, **characterized in that** for axial retainment there are provided two pins (16) extending transversally through the housing (4) and oriented roughly parallel with respect to each other, which extend in a direction roughly parallel to the swivel axis (3) and below which there engages a base part (18) arranged on the first sliding body (11), wherein the base part in one direction only has a maximum width (19) that corresponds to the distance (20) of the two pins (16) and in a direction roughly at 90° thereto has projections (21) engaging behind the pins (16).

6. Joint endoprosthesis according to one or several of claims 1 to 5, **characterized in that** the first sliding body (11) is provided with an undercut groove (24) that is open toward the opening of the housing (4) and that is formed by side walls oriented parallel with respect to each other, which groove (24) extends in the direction of the translatability of the second sliding body (22) relative to the first sliding body (11), and wherein the second sliding body (22) is provided with a spring (25) designed to correspond to the groove (24) and engages the groove (24) thereby.

7. Joint endoprosthesis according to claim 6, **characterized in that** the groove (24) extends transversally throughout the first sliding body (11) and that the spring (25) of the second sliding body (22), in the direction of the translatability of the second sliding body (22) relative to the first sliding body (11), has an extent which is shorter than the groove (24) by the extent of the translation path (27).

8. Joint endoprosthesis according to one or several of claims 1 to 7, **characterized in that** the first sliding body (11) is made from metal.

9. Joint endoprosthesis according to one or several of claims 1 to 8, **characterized in that** the housing (4) has a cylindrical portion (7) of the interior space, said portion extending inward from the opening of the interior space, and adjoining the same in the direction of the bottom of the housing has a conical portion (8), in particular a frustoconical portion (8).

10. Joint endoprosthesis according to one or several of claims 1 to 9, **characterized in that** the housing (4) at its end that extends into the bone has a supporting anchor (9) for insertion into the medullary cavity, to which preferably an extension anchor can be affixed.

11. Joint endoprosthesis according to one or several of claims 1 to 10, **characterized in that** the housing (4) is made from metal.

12. Joint endoprosthesis according to one or several of claims 1 to 11, **characterized in that** the housing (4) on its side that faces away from the first bone is provided with a sliding plate (10) projecting radially outward from the housing.

13. Joint endoprosthesis according to claim 12, **characterized in that** the second sliding body (22) is provided with a sliding plate (23) designed so as to have dimensions roughly equal to those of the housing (4) and by that sliding plate (23) rests against the sliding plate (10) of the housing (4).

14. Joint endoprosthesis according to claim 12 or 13, **characterized in that** both the sliding plate (10) of the housing (4) and the sliding plate (23) of the second articulation body (22) are of reniform design, with the longitudinal extent of the sliding plate (10) of the housing (4) extending roughly in the direction of the swivel axis (3).

15. Joint endoprosthesis according to one or several of claims 1 to 14, **characterized in that** the second sliding body (22) is made from plastics material, in particular from polyethylene.

16. Joint endoprosthesis according to one or several of claims 1 to 15, **characterized in that** the housing (4) can be anchored in the head of the tibia.

17. Joint endoprosthesis according to one or several of claims 1 to 16, **characterized in that** the first implant component (1) has structured surfaces on its external sides facing the bone.

18. Joint endoprosthesis according to claim 17, **characterized in that** the first implant component on that side of the sliding plate by which the latter rests against the epiphysis has a plurality of cylindrical depressions (10'), in particular bores (10'), with diameters of 0.3 to 1 mm, preferably 0.5 to 1 mm, and with depths between 0.3 and 1 mm, preferably 0.5 and 1 mm, such that a porosity of between 50 and 80 %, preferably 60 and 70 %, is realized (Fig. 11, 12).

19. Joint endoprosthesis according to claim 18, **characterized in that** in addition to the cylindrical depressions there are cylindrical protuberances (10") having diameters between 0.3 and 1 mm, preferably 0.5 and 1 mm, and heights between 0.3 and 1 mm, preferably 0.5 and 1 mm, with the ratio of the number of the cylindrical protuberances (10") to the number of the cylindrical depressions (10') amounting to 3 : 7 and with a porosity between 50 and 80 %, preferably 60 and 70 %, being present (Fig. 11, 12).

20. Joint endoprosthesis according to one or several of claims 17 to 19, **characterized in that** the first implant component (1) on the non-planar surfaces by which it is in contact with the bone has crater-like structured depressions with depths down to 0.5 mm (Fig. 13, 14).

## Revendications

1. Endoprothèse articulaire, en particulier prothèse de genou, susceptible d'être posée par une première partie de prothèse (1) sur une première extrémité d'un premier os, et par une deuxième partie de prothèse (2) sur une deuxième extrémité d'un deuxième os, des deux os formant une articulation, dans laquelle la première partie de prothèse (1) et la deuxième partie de prothèse (2) comportent chacune des surfaces articulaires pour former une articulation pivotante qui permet un pivotement autour d'un axe de pivotement (3) orienté approximativement perpendiculairement à l'extension longitudinale du premier et du deuxième os qui présentent la première et la deuxième extrémité, et comprenant en outre une partie coulissante (11, 22) qui permet, en supplément, un mouvement de rotation (D) et un mouvement de translation (V) des extrémités des os l'une par rapport à l'autre, ledit mouvement de rotation (D) ayant lieu autour d'un axe de rotation (15) approximativement perpendiculaire à l'axe de pivotement (3) et approximativement en direction longitudinale du premier os, et le mouvement de translation (V) ayant lieu dans un plan approximativement parallèle à l'axe de pivotement (3) et approximativement perpendiculairement à l'axe de rotation (15), la première partie de prothèse (1) comportant un boîtier (4) susceptible d'être enfoncé dans l'extrémité du premier os et susceptible d'être ancré dans cette extrémité du premier os, et dans le boîtier (4) est monté un premier corps coulissant (11), capable de tourner autour de l'axe de rotation (15), le deuxième corps coulissant (22) portant une surface articulaire (29) contre laquelle s'applique la deuxième partie de prothèse (2) au moyen de la surface articulaire ménagée sur celle-ci, et le deuxième corps coulissant (22) s'applique au moyen d'une surface de coulissement contre une surface de coulissement antagoniste prévue sur le boîtier (4), **caractérisée en ce que** :
- le deuxième corps coulissant (12) est monté dans le premier corps de coulissement (11) avec faculté de translation dans une direction approximativement perpendiculaire à l'axe de rotation (15),
- le deuxième corps coulissant (22) dépasse hors du boîtier (4), et
- la surface articulaire (29) du deuxième corps coulissant (22) est portée sur la partie dépassant hors du boîtier (4).

2. Endoprothèse articulaire selon la revendication 1, **caractérisée en ce que** le premier corps coulissant (11) est enfermé latéralement en totalité par le boîtier (4), et peut être mis en place dans le boîtier, ou respectivement enlevé hors du boîtier (4), à travers une ouverture du boîtier (4) détournée du premier os.

3. Endoprothèse articulaire selon l'une ou l'autre des revendications 1 et 2, **caractérisée en ce que** le premier corps de coulissement (11) est bloqué en direction de l'extension longitudinale du premier os dans le boîtier (4) à l'encontre d'un enlèvement hors du boîtier (4) dans la direction de l'axe de rotation (15).

4. Endoprothèse articulaire selon la revendication 3, **caractérisée en ce que** le blocage axial est réalisé au moyen d'un agencement à baïonnette (16, 18).

5. Endoprothèse articulaire selon la revendication 4, **caractérisée en ce que**, pour le blocage axial, il est prévu deux goupilles (16) s'étendant transversalement à travers le boîtier (4) et orientées approximativement parallèlement l'une à l'autre, lesdites goupilles s'étendant dans une direction approximativement parallèle à l'axe de pivotement (3), et sont engagées depuis le dessous par une partie de pied (18) agencée sur le premier corps coulissant (11), ladite partie de pied présentant dans une direction simplement une largeur maximale (19) qui correspond à la distance (20) des deux goupilles (16), et comporte des talons (21) dans une direction à environ 90° des goupilles et engageant lesdites goupilles (16) par l'arrière.

6. Endoprothèse articulaire selon l'une ou plusieurs des revendications 1 à 5, **caractérisée en ce que** le premier corps coulissant (11) est pourvu d'une gorge (24) en contre-dépouille et ouverte vers l'ouverture du boîtier (4), ladite gorge étant formée par des parois latérales orientées parallèlement l'une à l'autre, ladite gorge (24) s'étendant dans la direction de translation du deuxième corps coulissant (22) par rapport au premier corps coulissant (11), ledit deuxième corps coulissant (22) étant pourvu d'une languette (25) réalisée de façon correspondante à la gorge (24), et s'engageant de par cette languette dans la gorge (24).

7. Endoprothèse articulaire selon la revendication 6, **caractérisée en ce que** la gorge (24) s'étend transversalement à travers la totalité du premier corps coulissant (11), et **en ce que** la languette (25) du deuxième corps coulissant (22) présente une extension, en direction de translation du deuxième corps coulissant (22) par rapport au premier corps coulissant (11) plus courte que la gorge (24), de la valeur de la course de translation (27).

8. Endoprothèse articulaire selon l'une ou plusieurs des revendications 1 à 7, **caractérisée en ce que** le premier corps coulissant (11) est réalisé en métal.

9. Endoprothèse articulaire selon l'une ou plusieurs des revendications 1 à 8, **caractérisée en ce que** le boîtier (4) comprend une partie cylindrique (7) s'étendant vers l'intérieur depuis l'ouverture du volume intérieur, et une partie conique (8) en direction du fond du boîtier et se raccordant à ladite partie cylindrique, en particulier une partie de forme tronconique (8).

10. Endoprothèse articulaire selon l'une ou plusieurs des revendications 1 à 9, **caractérisée en ce que** le boîtier (4) comporte à son extrémité s'étendant dans l'os un ancrage de soutien (9) destiné à être mis en place dans le canal médullaire et sur lequel peut être de préférence fixé un ancrage de prolongement.

11. Endoprothèse articulaire selon l'une ou plusieurs des revendications 1 à 10, **caractérisée en ce que** le boîtier (4) est réalisé en métal.

12. Endoprothèse articulaire selon l'une ou plusieurs des revendications 1 à 11, **caractérisée en ce que**, du côté détourné du premier os, le boîtier (4) est pourvu d'une plaque coulissante (10) dépassant vers l'extérieur radialement depuis le boîtier.

13. Endoprothèse articulaire selon la revendication 12, **caractérisée en ce que** le deuxième corps coulissant (22) est pourvu d'une plaque coulissante (23), dont les dimensions sont approximativement égales à 1 celles du boîtier (4), et repose au moyen de cette plaque coulissante (23) contre la plaque coulissante (10) du boîtier (4).

14. Endoprothèse articulaire selon l'une ou l'autre des revendications 12 et 13, **caractérisée en ce que** la plaque coulissante (10) du boîtier (4) tout comme la plaque coulissante (23) du deuxième corps coulissant (22) sont conçues sous forme ovale, l'extension longitudinale de la plaque coulissante (10) du boîtier (4) étant dirigée approximativement en direction de l'axe de pivotement (3).

15. Endoprothèse articulaire selon l'une ou plusieurs des revendications 1 à 14, **caractérisée en ce que** le deuxième corps coulissant (22) est réalisé en matière plastique, en particulier en polyéthylène.

16. Endoprothèse articulaire selon l'une ou plusieurs des revendications 1 à 15, **caractérisée en ce que** le boîtier (4) est susceptible d'être ancré dans la tête du tibia.

17. Endoprothèse articulaire selon l'une ou plusieurs des revendications 1 à 16, **caractérisée en ce que** la première partie de prothèse (1) présente des surfaces structurées sur ses faces extérieures tournées vers l'os.

18. Endoprothèse articulaire selon la revendication 17, **caractérisée en ce que** la première partie de prothèse comporte, sur le côté de la plaque coulissante par lequel celle-ci s'applique contre l'extrémité de l'os, une pluralité de renfoncements cylindriques (10'), en particulier de perçages (10'), avec des diamètres de 0,3 à 1 mm, de préférence de 0,5 à 1 mm, et avec des profondeurs entre 0,3 et 1 mm, de préférence entre 0,5 et 1 mm, de sorte qu'il en résulte une porosité entre 50 et 80 %, de préférence entre 60 et 70 % (figures 11 et 12).

19. Endoprothèse articulaire selon la revendication 18, **caractérisée en ce que**, en supplément aux renfoncements cylindriques, il est prévu des saillies cylindriques (10") avec des diamètres compris entre 0,3 et 1 mm, de préférence entre 0,5 et 1 mm, et des hauteurs comprises entre 0,3 et 1 mm, de préférence entre 0,5 et 1 mm, le rapport entre le nombre des saillies cylindriques (10") et le nombre des renfoncements cylindriques (10') s'élevant à 3:7, et il en résulte une porosité entre 50 et 80 %, de préférence entre 60 et 70 % (figures 11 et 12).

20. Endoprothèse articulaire selon l'une ou plusieurs des revendications 17 à 19, **caractérisée en ce que** la première partie de prothèse comporte, au niveau des surfaces non planes via lesquelles elle est en contact avec l'os, des renfoncements structurés en forme de cratère avec des profondeurs allant jusqu'à 0,5 mm (figures 13 et 14).
